# EUROPEAN PATENT SPECIFICATION

(11) **EP 2 129 421 B1**
(45) Date of publication and mention of the grant of the patent: **05.01.2011**
(21) Application number: 07827703.5
(22) Date of filing: 19.09.2007
(51) Int. Cl.: A61M 15/00

(54) **Improved metered dose inhaler for aerosol**
Verbesserter Dosierinhalator für Aerosol
Inhalateur perfectionné à dose mesurée pour un aérosol

(30) Priority: 27.03.2007 IT MI20070612
(43) Date of publication of application: 09.12.2009
(73) Proprietor: Rizzi, Annamaria, 22036 Erba (IT)
(72) Inventor: Rizzi, Annamaria, 22036 Erba (IT)
(74) Representative: Adorno, Silvano
(86) International application number: PCT/IT2007/000652
(87) International publication number: WO 2008/117322

(56) References cited:
- GB-A- 2 230 456
- US-A- 4 796 614
- US-A- 4 852 561
- US-A- 5 040 527

## Description

The present invention relates to an improved metered dose inhaler for aerosol.

Asthma is an extremely common pathology characterized by a difficult air flow through the bronchial tubes, particularly during the exhalation phase. This results in cough as well as shortness of breath for the patient.

Asthma diseases require a continuous treatment consisting in making the patient take drugs through aerosol on a regular base. In such a way, the inflammation condition of the air ducts can be kept under control without resulting in the so-called bronchial hyperactivity. The treatments for the asthma diseases last throughout the patient's life.

A type of very diffused devices for the treatment of asthma diseases are metered dose inhalers, also known under the acronym MDI. Such devices usually comprise a container under pressure, provided with a delivery valve and containing a drug in the liquid state, and an actuator provided with an inhalation mouthpiece. The container is generally vertically inserted into the actuator so that the valve enters into an axial seat suitably arranged at the bottom of the actuator. The seat housing the container valve is provided with a transversally arranged nozzle whose outlet faces the actuator mouthpiece. By pushing the container towards the inside of the actuator, the valve delivers a metered dose of drug that is deviated by the nozzle towards the mouthpiece in order to be inhaled by the patient.

Although metered dose inhalers are extremely practical, inexpensive and not very cumbersome, it is known that the percentage of drug actually reaching the bronchial tubes of a patient is only about 30% of the dose delivered by the container. This is mainly due to the fact that metered dose inhalers require a good coordination between the step of delivery of the drug from the container and the inspiratory step enabling to inhale the drug, which coordination a patient, especially a baby, can not always achieve especially during an asthma crisis. Moreover, the pressure of the spray delivered through the mouthpiece causes the drug to mainly deposit onto the oropharyngeal duct, thus reaching the bronchial tubes, which are the real target of the drug, in a lower percentage only.

It has been known for many years the use of auxiliary devices, known as "spacers", to be applied to metered dose inhalers in order to solve these drawbacks. These devices are essentially tubes of suitable diameter and length that are inserted at one end onto the mouthpiece of the inhaler and exhibit at the opposite end a second mouthpiece, which actually enables the delivery of the drug and is provided with a unidirectional valve. By pushing on the container the drug is delivered into the spacer, where it stays until it is inhaled later, rather than into the oral duct of the patient. Thus, spacer devices eliminate the need for coordinating the inspiratory act with the step of delivery of the drug. Further, the unidirectional valve of the spacer is triggered by the patient only when a given depression threshold is exceeded, thus enabling the drug to reach the bronchial tubes more in depth. The result of using a spacer combined with metered dose inhaler is that higher percentages of drug reach the bronchial tubes, thus improving the efficacy of the treatment and minimizing the waste of drug.

An example of spacer is given by British patent GB 2230456 to Glaxo Group Limited, wherein an inhalation chamber, particularly suited for babies, is described to be used with a metered dose inhaler for aerosol. The chamber is provided with an inlet suitable for housing the mouthpiece of a metered does inhaler and with an outlet provided with a unidirectional valve and a mask for the face. The patent underlines the importance of the volume of the spacer chamber, which has to be preferably 5 to 15 times greater than the inhalation respiratory volume of a patient in order to make the greater amount of drug as possible reach the mouthpiece of the device. The length of the spacer chamber also contributes to maximize the amount of drug reaching the mouthpiece and thus the outflow efficiency, therefore the teaching of the patent allows to correctly dimension a spacer in function of the kind of patient who will use it.

The market of aerosol products offers a complete range of sizes of spacers to be applied to a metered dose inhaler, however they have the big disadvantage of being auxiliary devices to be added to the inhaler and having to be chosen on the basis of the age and the inspiratory capacity of the patient. In addition, spacers are quite expensive and cumbersome, thus almost completely eliminating the advantages of compactness, transportability and inexpensiveness of metered dose inhalers.

Another drawback of the spacers is that they must be inserted onto the mouthpiece of metered dose inhalers, resulting in potential problems of loss of the drug delivered.

Patent US 4852561 discloses an inhalation device comprising a housing that defines an aerosol chamber for receiving a metered dose of a medicine containing aerosol from a cartridge. The cartridge is wholly supported within the aerosol chamber. An actuating valve mechanism is slidingly receivable within an open end of the housing and includes a projecting portion to which a compressive force may be applied. Upon application of the compressive force, the outlet valve mechanism of the cartridge is activated to dispense a metered dose of medicine into the aerosol chamber in the form of an aerosol. The actuating valve mechanism is provided with openings allowing the ingress of air to prevent the formation of partial vacuum within the aerosol chamber during inhalation.

The above-mentioned patent describes a solution in which a chamber, i.e. the spacer, is integrated in the device, thus allowing to achieve a compact device suitable to be carried about e.g. in a jacket pocket. However, the chamber is not airtight due to the openings formed at the end opposite to the mouthpiece. Hence, in order not to lose a part of the metered dose of the medicine, the user must inhale substantially immediately after the delivery phase, thus partially losing the benefit of having a chamber integrated in the device.

Patent US 4796614 discloses a collapsible inhalation valve for administering asthmatic medication or the like. The valve comprises a cylindrical air chamber adapted to be held in the hand. A reciprocating member holds a pressurized cartridge of medication for reciprocation between a storage position substantially within the air chamber and a raised using position substantially exterior to the air chamber. A mouthpiece is pivoted on the air chamber and is movable between a storage position substantially within the air chamber and a using position projecting from the air chamber. A one-way valve is provided at the bottom of the air chamber to allow movement of air into the air chamber while preventing movement of medication out of the air chamber. Another one-way valve is provided at the entrance to the mouthpiece permitting inhaling of misted medication, but preventing exhaling into the air chamber.

The above-mentioned patent describes a solution in which a chamber is integrated in the device and closed by means of one-way valves, thus allowing to achieve a more compact device and to retain the dose of drug inside the spacer chamber after delivery. However, the collapsible structure of the device is very complex and in order to use the inhaler the user must first withdraw the cartridge and then rotate the mouthpiece, which might be difficult in particular during an asthma crisis.

It is therefore an object of the present invention to provide an improved metered dose inhaler, which is free from said disadvantages. Said object is achieved with a metered dose inhaler, whose main features are disclosed in the first claim, while other features are disclosed in the remaining claims.

The improved metered dose inhaler according to the present invention comprises an actuator inside which a chamber functioning as a spacer is formed, receiving and retaining the drug delivered by the container under pressure. The chamber is normally tightly closed by means of a first unidirectional closing valve arranged at the end of the chamber that is in proximity to the inhalation mouthpiece, and by means of one or more second unidirectional closing valves arranged at the opposite end of the chamber in correspondence to a plurality of apertures formed into the top surface of the actuator. The opening of the first unidirectional closing valve determines the opening of said one or more second unidirectional closing valves, thus enabling an inflow of air passing throughout the chamber from one end to the other favoring the outflow of the dose of drug.

The main advantage of the inhaler according to the present invention is that, thanks to the integration of the spacer in the actuator body, a patient carries with him one single inhaler-spacer device whose size is strongly reduced with respect to the inhaler-spacer assembly of the prior art.

Consequently, the drug loss problems during the step of delivery of the drug caused by the insertion of the spacers onto the mouthpiece of the metered dose inhalers of the prior art are completely eliminated and the manufacturing costs are extremely reduced, resulting in potential competitive advantages in the market of portable devices for aerosol.

Another advantage offered by the invention is that, thanks to the opening of said one or more unidirectional valves, the volume of the chamber functioning as a spacers is virtually infinite during the inhalation step and thus capable of adapting to the inspiratory capacity of any user, being either an adult or a baby, allowing to solve the technical prejudice of being obliged to have an accumulation volume for the specific type and age of the patient.

Moreover, the air flow passing throughout the chamber from one end to the other enables the complete outflow of the dose of drug, avoiding rests to remain inside the inhaler.

Further advantages and features of the improved metered dose inhaler according to the present invention will become clear to those skilled in the art from the following detailed and non-limiting description of an embodiment thereof with reference to the attached drawings, wherein:
- figure 1 shows a perspective view of an aerosol inhaler;
- figure 2 shows a top view of the aerosol inhaler of figure 1;
- figure 3 shows a longitudinal sectional view along line III-III of figure 2;
- figure 4 shows a detail IV of figure 3;
- figure 5 shows a perspective view of the inhaler according to the invention;
- figure 6 shows a top view of the inhaler of figure 5;
- figure 7 shows a longitudinal sectional view VII-VII of figure 6;
- figure 8 shows a detail VIII of figure 7; and
- figure 9 shows a perspective view of the assembly of the first and second unidirectional closing valves.

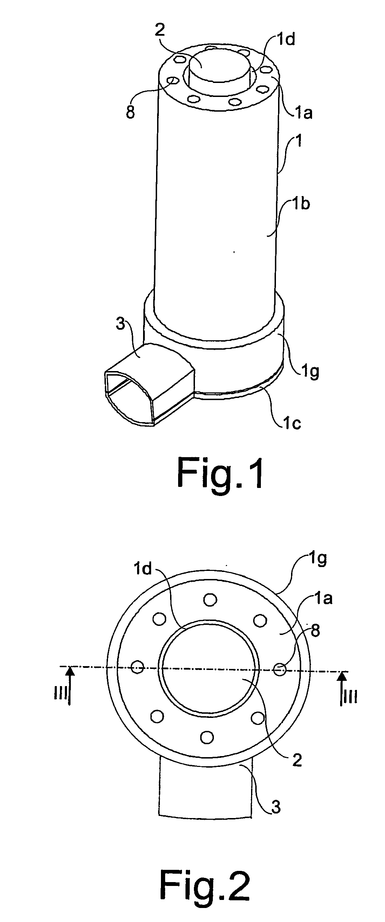

Referring to figures 1 and 2, the metered dose inhaler for aerosol comprises in a known way an actuator 1 formed of a top surface 1a, a side wall 1b and a lower lid 1c. Actuator 1 is provided with a cavity 1d suitable for housing a common metered dose container 2 for aerosol provided with a delivery valve 2a. Actuator 1 is further provided with a mouthpiece 3 arranged downstream of cavity 1d and suitable to enable a user to inhale a metered dose of drug delivered by container 2.

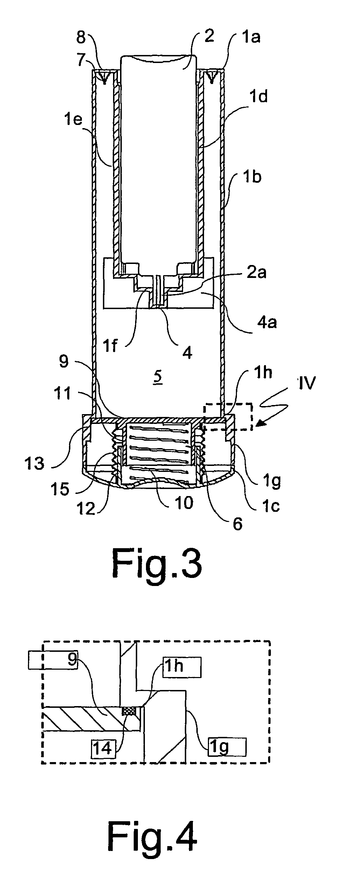

As shown in figures 3 and 4, cavity 1d of the inhaler is substantially coaxially arranged relative to side wall 1b and is in turn provided with a side wall 1e and a bottom 1f wherein a nozzle 4 is formed suitable for seating delivery valve 2a of container 2 in order to allow a metered dose of drug to be delivered. Nozzle 4 communicates with a chamber 5 defined inside actuator 1 and chamber 5 is normally tightly closed at one end by means of a first unidirectional closing valve 6 arranged in proximity to mouthpiece 3 and at the opposite end by means of one or more second unidirectional closing valves 7 arranged in correspondence to one or more apertures 8 formed therein.

During the operation of the inhaler, container 2 is pressed towards the inside of actuator 1 thus delivering a metered dose of drug. The dose of drug enters chamber 5 through nozzle 4 and is kept therein by unidirectional closing valves 6, 7. Subsequently, the inspiratory act of a patient causes the opening of first unidirectional closing valve 6. This generates a depression inside chamber 5, which activates a flow of the dose of drug towards mouthpiece 3 and at the same time determines the opening of the one or more second unidirectional closing valves 7, thus generating an air flow inside chamber 5, which passes throughout it from one end to the other, i.e. from top surface 1a to first unidirectional closing valve 6, favoring the outflow of the dose of drug through mouthpiece 3.

Chamber 5 has a volume comprised between 5 and 100 ml, necessary to grant enough space to receive the dose of drug delivered by container 2. The opening of the one or more unidirectional valves 7 makes virtually infinite the volume of chamber 5 and thus suitable to any pulmonary capacity, thus allowing to accomplish one single size of inhaler for any kind of patient.

Cavity 1d is preferably connected to side wall 1b of actuator 1 by means of a plurality of radial ribs 4a suitable for withstanding the compression force of container 2 needed for triggering valve 2a during the delivery of a dose of drug. Preferably, radial ribs 4a are arranged in correspondence to bottom 1f of cavity 1d.

In proximity to mouthpiece 3 actuator 1 is provided with a portion 1g of larger diameter that houses first unidirectional closing valve 6. First unidirectional closing valve 6 comprises a plate 9 arranged in abutment on a shoulder 1h defined at larger diameter portion 1g of actuator 1. Plate 9 is pressed on shoulder 1h by means of a spring 10 arranged between lower lid 1c and plate 9 itself.

In order to allow a correct opening and closing movement in the axial direction of first unidirectional closing valve 6, plate 9 is provided with a hollow stem 11 wherein spring 10 is inserted, and hollow stem 11 is in turn slidably inserted in a cylindrical sleeve 12 integral with lower lid 1c.

In addition, actuator 1 preferably comprises a series of longitudinal ribs 13 arranged on the inner periphery of larger diameter portion 1g, which help in guiding plate 9 along the axial direction.

In order to ensure a good sealing action of the drug inside chamber 5, an annular sealing element 14, e.g. made of silicon rubber, is preferably arranged between plate 9 and shoulder 1h.

In order to prevent the drug to meander between hollow stem 11 and cylindrical sleeve 12, a protection element 15 is provided, which is tightly inserted with one of its ends on hollow stem 11 of plate 9 and with the other end on cylindrical sleeve 12 of lower lid 1c. In order to follow the opening and closing movements of first unidirectional closing valve 6, protection element 15 may be, for instance, a flexible bellows element.

In figures 1 to 4, a plurality of second unidirectional closing valves 7 are shown, e.g. of a lamellar type, being each arranged in correspondence to an aperture 8 and opening towards the inside of chamber 5 upon the depression generated in the chamber at the opening of first unidirectional valve 6.

Alternatively, it is possible to provide second unidirectional closing valves 7 by means of a single annular membrane tightly fixed on apertures 8 and provided with a series of lip slits, being each arranged is correspondence to an aperture 8, which, similarly to lamellar valves, open towards the inside of chamber 5 upon the depression generated in the chamber at the opening of first unidirectional valve 6.

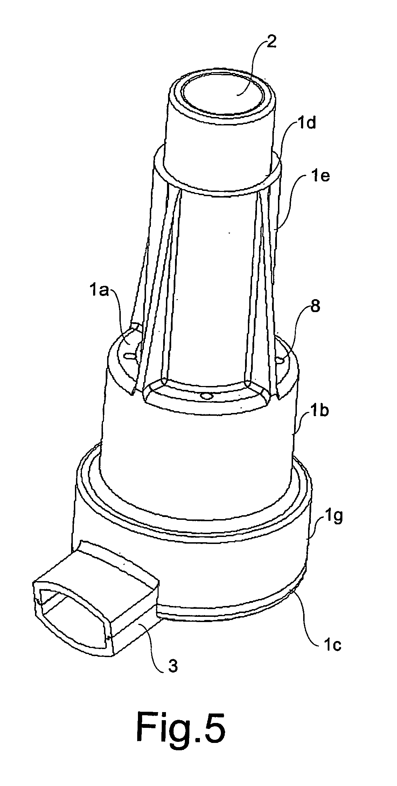

Figures 5 to 8 show the metered dose inhaler for aerosol according to the present invention, wherein the structure of actuator 1 has been optimized in view of manufacturing needs. Top surface 1a is now at the base of side wall 1e surrounding cavity 1d, thus clearly separating the seat of container 2 from chamber 5. Side wall 1 e has been suitably stiffened by means of a series of radial ribs.

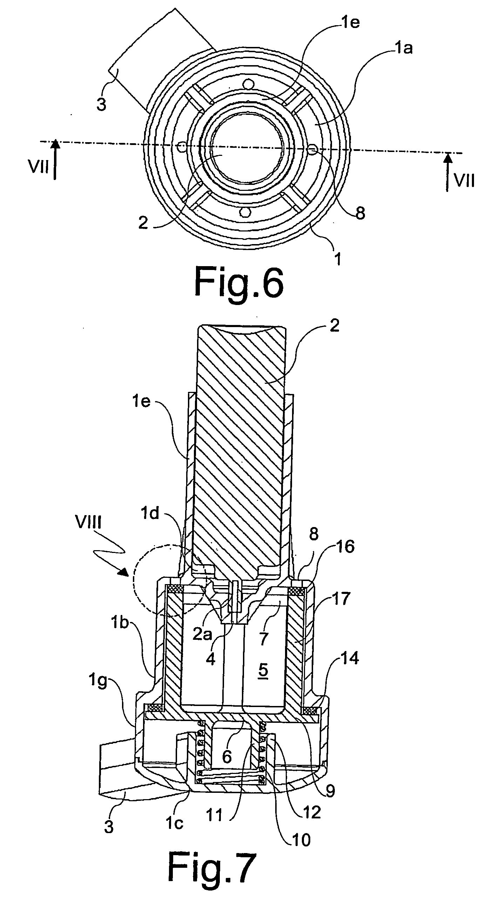

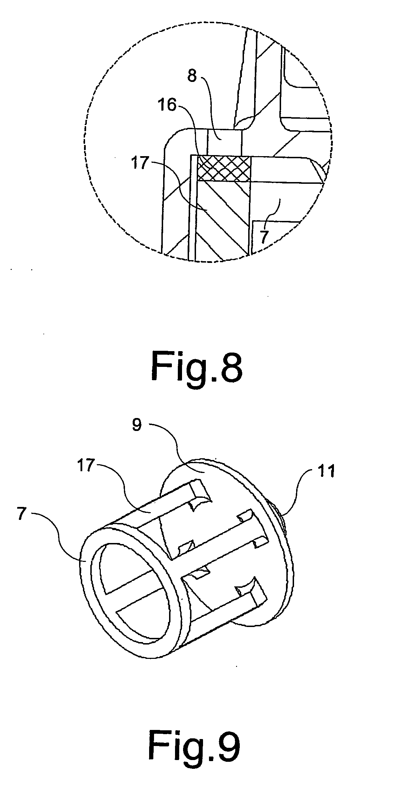

According to this embodiment, as shown in figures 7 and 8, apertures 8 are closed by means of a single second unidirectional closing valve 7, having an annular shape and being provided with a sealing element 16, e.g. a silicon ring, arranged on the surface contacting apertures 8.

Second unidirectional closing valve 7 is rigidly connected in the axial direction to plate 9 of first unidirectional closing valve 6 through a series of mountings 17. Thus, upon the inspiratory act of a patient, the movement of first closing valve 6 will immediately determine the movement of second closing valve 7, resulting in the opening of chamber 5 in correspondence to its ends at the same time in order to enable the outflow of the drug. This solution is particularly advantageous as the absence of a time delay in the opening of the second closing valve with respect to the first one allows to immediately reach the ideal outflow conditions for the drug.

Possible modifications and/or additions may be made by those skilled in the art to the hereinabove disclosed and illustrated embodiments while remaining within the scope of the following claims.

## Claims

1. A metered dose inhaler comprising an actuator (1) provided with a cavity (1d), suitable for housing a metered dose container (2) for aerosol provided with a delivery valve (2a), and with a mouthpiece (3) arranged downstream of said cavity and suitable for enabling a user to inhale a metered dose of drug delivered by said container (2), said cavity (1d) being provided with a bottom (1f) wherein a nozzle (4) is formed suitable for housing the delivery valve (2a) of said container (2) in order to enable the delivery of a metered dose of drug and said nozzle (4) communicating with a chamber (5) defined inside the actuator (1), said chamber (5) being tightly closed at one end by means of a first unidirectional closing valve (6) arranged in proximity to the mouthpiece (3) and at the opposite end by means of a second unidirectional closing valves (7) arranged in correspondence to a plurality of apertures (8) formed therein, **characterized in that** said second unidirectional closing valve (7) is rigidly connected in the axial direction to a plate (9) of said first unidirectional closing valve (6) through a series of mountings (17), such that the movement of the first unidirectional closing valve (6) upon the inspiratory act of a patient immediately determines the movement of the second unidirectional closing valve (7), thus resulting in the simultaneous opening of the chamber (5) in correspondence to its ends and **in that** the second unidirectional closing valve (7) is a single unidirectional closing valve having an annular shape.

2. An inhaler according to claim 1, **characterized in that** the volume of the chamber (5) of the actuator (1) is comprised between 5 and 100 ml.

3. An inhaler according to claim 1, **characterized in that** said actuator (1) is provided with a portion (1g) of larger diameter in proximity to the mouthpiece (3), suitable for housing said first unidirectional closing valve (6).

4. An inhaler according to claim 3, **characterized in that** said plate (9) is arranged in abutment on a shoulder (1h) defined in correspondence to said larger diameter portion (1g) of the actuator (1), said plate (9) being pressed on said shoulder (1h) by a spring (10) arranged between the plate (9) and a lower lid (1c) of the actuator (1).

5. An inhaler according to claim 4, **characterized in that** between the plate (9) and the shoulder (1h) an annular sealing element (14) is arranged.

6. An inhaler according to claim 4 or 5, **characterized in that** said plate (9) is provided with a hollow stem (11) wherein said spring (10) is inserted, said hollow stem (11) being slidably inserted in a cylindrical sleeve (12) integral with the lower lid (1c) of the actuator (1).

7. An inhaler according to claim 6, **characterized by** further comprising a protection element (15) tightly inserted on the hollow stem (11) with one of its ends and on the cylindrical sleeve (12) with its other end, said protection element (15) being an elastic bellows suitable for following the opening and closing movements of the plate (9) of the first unidirectional closing valve (6).

8. An inhaler according to claim 1, **characterized in that** said second unidirectional closing valve (7) is provided with a sealing element (16) arranged on the surface contacting the apertures (8).

## Patentansprüche

1. Ein Dosierinhalator mit einer Auslösevorrichtung (1), welche mit einer Aussparung (1d) versehen ist, die geeignet ist zur Aufnahme eines Behälters (2) für eine abgemessene Aerosoldosis samt einem Zuführventil (2a), und mit einem Mundstück (3), welches stromabwärts der besagten Aussparung (1d) angeordnet und geeignet ist, den Benutzer zur Inhalation einer abgemessenen Dosis des Medikaments zu befähigen, welches durch besagten Behälter (2) zur Verfügung gestellt wird, wobei die besagte Aussparung (1d) mit einem Boden (1f) versehen ist, worin eine Düse (4) ausgeformt ist, die zur Aufnahme des Zuführventils (2a) des besagten Behälters (2) geeignet ist, um die Zufuhr einer abgemessenen Medikamentendosis zu ermöglichen, und wobei die besagte Düse (4) mit einer Kammer (5) kommuniziert, die innerhalb der Auslösevorrichtung (1) abgegrenzt ist, wobei die besagte Kammer (5) an einem Ende mit einem ersten Rückschlagventil (6) dicht verschlossen ist, welches in der Nähe des Mundstücks (3) angeordnet ist, sowie am gegenüberliegendem Ende durch ein zweites Rückschlagventil (7), welches mit einer Vielzahl von darin eingeformten Öffnungen (8) korrespondierend angeordnet ist, **dadurch gekennzeichnet, dass** das besagte zweite Rückschlagventil (7) in axialer Richtung mittels einer Reihe von Halterungen (17) fest mit einer Platte (9) des besagten ersten Rückschlagventils (6) verbunden ist, derart, dass die Bewegung des ersten Rückschlagventils (6) anläßlich des Einatmens des Patienten unverzüglich die Bewegung des zweiten Rückschlagventils (7) bestimmt, wodurch eine gleichzeitige Öffnung der Kammer (5) korrespondierend mit ihren Enden resultiert, sowie **dadurch**, dass das zweite Rückschlagventil (7) ein einzelnes Rückschlagventil von ringförmiger Gestalt ist.

2. Ein Inhalator nach Anspruch 1, **dadurch gekennzeichnet, dass** das Volumen der Kammer (5) der Auslösevorrichtung (2) sich in einem Bereich von 5 bis 100 ml befindet.

3. Ein Inhalator nach Anspruch 1, **dadurch gekennzeichnet, dass** die besagte Auslösevorrichtung (2) in der Nähe des Mundstücks (3) mit einem Bereich (1 g) von größerem Durchmesser versehen ist, der geeignet ist zur Aufnahme des besagten ersten Rückschlagventils (6).

4. Ein Inhalator nach Anspruch 3, **dadurch gekennzeichnet, dass** besagte Platte (9) an einer Schulter (1h) anliegt, die an dem Bereich größeren Durchmessers (1 g) der Auslösevorrichtung definiert ist, wobei die besagte Platte (9) auf die besagte Schulter gepresst wird mittels einer Feder (10), die zwischen der Platte (9) und einem unteren Deckel (1c) der Auslösevorrrichtung (1) angeordnet ist.

5. Ein Inhalator nach Anspruch 4, **dadurch gekennzeichnet, dass** zwischen der Platte (9) und der Schulter (1h) ein ringförmiges Dichtelement (14) angeordnet ist.

6. Ein Inhalator nach Anspruch 4 oder 5, **dadurch gekennzeichnet, dass** die besagte Platte (9) mit einem hohlen Stempel (11) versehen ist, worin die besagte Feder (10) eingelassen ist, wobei besagter hohler Stempel verschiebbar in eine zylindrische Hülle (12) eingeschoben ist, die mit dem unteren Deckel (1c) der Auslösevorrichtung (1) integriert ist.

7. Ein Inhalator nach Anspruch 6, weiterhin **gekennzeichnet durch** ein Schutzelement (15), welches mit einem seiner Enden abdichtend in den hohlen Stempel (11) eingefügt ist und mit seinem anderen Ende auf den zylindrischen Mantel (12) aufgesetzt ist, wobei das besagte Schutzelement (15) ein elastischer Blasebalg ist, der geeignet ist, den Öffnungs- und Verschlussbewegungen der Platte (9) des ersten Rückschlagventils (6) zu folgen.

8. Inhalator nach Anspruch 1, **dadurch gekennzeichnet, dass** das zweite Rückschlagventil (7) mit einem Dichtelement (16) versehen ist, welches auf derjenigen Fläche angebracht ist, die mit den Öffnungen (8) in Kontakt steht.

## Revendications

1. Inhalateur à dose mesurée comprenant un actionneur (1) prévu avec une cavité (1 d), appropriée pour loger un récipient à dose mesurée (2) pour aérosol prévu avec une valve de distribution (2a) et avec un embout buccal (3) agencé en aval de ladite cavité et approprié pour permettre à un utilisateur d'inhaler une dose mesurée de médicament distribué par ledit récipient (2), ladite cavité (1d) étant prévue avec un fond (1f) dans lequel une buse (4) est formée, appropriée pour loger la valve de distribution (2a) dudit récipient (2) afin de permettre la distribution d'une dose mesurée de médicament et ladite buse (4) communiquant avec une chambre (5) définie à l'intérieur de l'actionneur (1), ladite chambre (5) étant hermétiquement fermée au niveau d'une extrémité au moyen d'un premier clapet de fermeture unidirectionnel (6) agencé à proximité de l'embout buccal (3) et au niveau de l'extrémité opposée au moyen d'un deuxième clapet de fermeture unidirectionnel (7) agencé en correspondance avec une pluralité d'ouvertures (8) formées à l'intérieur de cette dernière, **caractérisé en ce que** ledit deuxième clapet de fermeture unidirectionnel (7) est rigidement raccordé dans la direction axiale à une plaque (9) dudit premier clapet de fermeture unidirectionnel (6) par le biais d'une série de montages (17), de sorte que le mouvement du premier clapet de fermeture unidirectionnel (6), suite à l'acte inspiratoire d'un patient, détermine immédiatement le mouvement du deuxième clapet de fermeture unidirectionnel (7), se traduisant ainsi par l'ouverture simultanée de la chambre (5) en correspondance avec ses extrémités et **en ce que** le deuxième clapet de fermeture unidirectionnel (7) est un clapet de fermeture unidirectionnel unique ayant une forme annulaire.

2. Inhalateur selon la revendication 1, **caractérisé en ce que** le volume de la chambre (5) de l'actionneur (1) est compris entre 5 et 100 ml.

3. Inhalateur selon la revendication 1, **caractérisé en ce que** ledit actionneur (1) est prévu avec une partie (1g) de plus grand diamètre à proximité de l'embout buccal (3), approprié pour loger ledit premier clapet de fermeture unidirectionnel (6).

4. Inhalateur selon la revendication 3, **caractérisé en ce que** ladite plaque (9) est agencée en butée sur un épaulement (1 h) défini en correspondance avec ladite partie de plus grand diamètre (1g) de l'actionneur (1), ladite plaque (9) étant comprimée sur ledit épaulement (1h) par un ressort (10) agencé entre la plaque (9) et un couvercle inférieur (1c) de l'actionneur (1).

5. Inhalateur selon la revendication 4, **caractérisé en ce qu'**entre la plaque (9) et l'épaulement (1h), on agence un élément d'étanchéité annulaire (14).

6. Inhalateur selon la revendication 4 ou 5, **caractérisé en ce que** ladite plaque (9) est prévue avec une tige creuse (11) dans laquelle ledit ressort (10) est inséré, ladite tige creuse (11) étant insérée de manière coulissante dans un manchon cylindrique (12) solidaire du couvercle inférieur (1c) de l'actionneur (1).

7. Inhalateur selon la revendication 6, **caractérisé en ce qu'**il comprend en outre un élément de protection (15) inséré de manière étanche sur la tige creuse (11) avec l'une de ses extrémités et sur le manchon cylindrique (12) avec son autre extrémité, ledit élément de protection (15) étant un soufflet élastique approprié pour suivre les mouvements d'ouverture et de fermeture de la plaque (9) du premier clapet de fermeture unidirectionnel (6).

8. Inhalateur selon la revendication 1, **caractérisé en ce que** ladite deuxième clapet de fermeture unidirectionnel (7) est prévu avec un élément d'étanchéité (16) agencé sur la surface en contact avec les ouvertures (8).
